# EUROPEAN PATENT APPLICATION

(11) **EP 4 230 124 A1**
(43) Date of publication of application: **23.08.2023**
(21) Application number: 21881635.3
(22) Date of filing: 30.07.2021
(51) Int. Cl.: A61B 5/021

(54) **BLOOD PRESSURE MEASUREMENT MODULE, WATCH STRAP FOR WEARABLE DEVICE, AND WEARABLE DEVICE**

(30) Priority: 22.10.2020 CN 202022374440 U; 22.10.2020 CN 202011139126
(71) Applicant: GUANGDONG OPPO MOBILE TELECOMMUNICATIONS CORP., LTD., Dongguan, Guangdong 523860 (CN)
(72) Inventor: ZHOU, Shun, Dongguan, Guangdong 523860 (CN)
(74) Representative: Penza, Giancarlo
(86) International application number: PCT/CN2021/109766
(87) International publication number: WO 2022/083215

(57) **Abstract**

Provided are a blood pressure measurement module (2), a watch strap (100) for a wearable device, and the wearable device having same. The blood pressure measurement module (2) comprises a housing (21), an emitting element (23), a light receiver (24), and a circuit board (22). The housing (21) is provided with a transparent region. The emitting element (23) and the light receiver (24) are both provided on the circuit board (22). The circuit board (22) is provided in an accommodating space. The emitting element (23) is used for emitting infrared light to a finger of a user by means of the transparent region. The light receiver (24) is electrically connected to the emitting element (23). The light receiver (24) is used for receiving, by means of the transparent region, the infrared light reflected by the finger of the user. The circuit board (22) is used for calculating a blood pressure value according to the reflected infrared light received by the light receiver (24).

## Description

### CROSS-REFERENCE TO RELATED APPLICATIONS

The application is based on Chinese patent applications with application numbers of 202022374440.7 and 202011139126.9 and the application date of October 22, 2020, and claims priority to the Chinese patent applications. The entire contents of the Chinese patent applications are hereby incorporated herein by reference.

### TECHNICAL FIELD

The present disclosure relates to the field of electronic devices, and in particularly, to a blood pressure measurement module, a watch strap for wearable devices and a wearable device.

### BACKGROUND

With the development of society, people pay increasing attention to their own health, and wearable devices with blood pressure measurement function (such as smart watches, smart bands, etc.) also receive increasing attention.

### SUMMARY

One objective of the present disclosure is to propose a blood pressure measurement module, which can improve the accuracy of blood pressure measurement.

The present disclosure further proposes a watch strap for wearable devices, the watch strap includes the blood pressure measurement module.

The present disclosure further proposes a wearable device with the watch strap.

In a first aspect, an embodiment of the present disclosure proposes a blood pressure measurement module including: a housing provided with a holding space therein and a transparent area thereon, an emission element, a light receiver and a circuit board; the emission element and the light receiver are all disposed on the circuit board, the circuit board is disposed in the holding space, the emission element is configured to transmit infrared light to a user's finger through the transparent area, the light receiver is electrically connected with the emission element, the light receiver is configured to receive infrared light reflected by the user's finger through the transparent area, the circuit board is configured to calculate a blood pressure value according to the reflected infrared light received by the light receiver.

In a second aspect, an embodiment of the present disclosure proposes a watch strap for wearable devices including: a strap body provided with an installation groove; and the blood pressure measurement module according to the above embodiment of the present disclosure, which is installed in the installation groove.

In a third aspect, an embodiment of the disclosure proposes a wearable device including: a watch body, and the watch strap according to the above embodiment of the second aspect of the present disclosure; and the watch strap is connected with the watch body.

Additional aspects and advantages of the present disclosure will be given in the following description, and some will become obvious from the following description, or learned from the practice of the present disclosure.

### BRIEF DESCRIPTION OF THE DRAWINGS

FIG. 1 illustrates a perspective view of a blood pressure measurement module according to some embodiments of the present disclosure.
FIG. 2 illustrates an exploded view of the blood pressure measurement module according to some embodiments of the present disclosure.
FIG. 3 illustrates an exploded view of a first housing of the blood pressure measurement module according to some embodiments of the present disclosure.
FIG. 4 illustrates a schematic diagram of an assembly of a second housing, a circuit board and a power supply of the blood pressure measurement module according to some embodiments of the present disclosure.
FIG. 5 illustrates a schematic diagram of a second housing of the blood pressure measurement module according to some embodiments of the present disclosure.
FIG. 6 illustrates a perspective view of a watch strap for wearable devices according to some embodiments of the present disclosure.
FIG. 7 illustrates an exploded view of a watch strap for wearable devices according to some embodiments of the present disclosure.
FIG. 8 illustrates another exploded view of a watch strap for wearable devices according to some embodiments of the present disclosure.

Description of reference numerals:
100, watch strap;
1, strap body; 11, installation groove;
2, blood pressure measurement module; 21, housing; 211, first housing; 2111, first part; 2112, second part; 212, second housing; 2121, partition plate; a, first space; b, second space; 213, first positioning structure; 214, through groove; 215, inlaying groove; 215a, first sub-groove; 215b, second sub-groove; 2151, positioning groove; 22, circuit board; 221, second positioning structure; 23, emission element; 24, light receiver; 25, lens; 251, first lens; 252, second lens; 253. positioning protrusion; 26, power supply; 27, charging connector; 28, communication circuit; 29. resilient sheet.

### DETAILED DESCRIPTION OF EMBODIMENTS

The embodiments of the present disclosure are described in detail below. The examples of the embodiments are shown in the attached drawings, where the same or similar labels represent the same or similar elements or elements with the same or similar functions from beginning to end. The embodiments described below with reference to the attached drawings are illustrative and are only used to explain the present disclosure and cannot be understood as restrictions on the present disclosure.

A blood pressure measurement module 2 according to an embodiment of the present disclosure is described below with reference to the attached drawings.

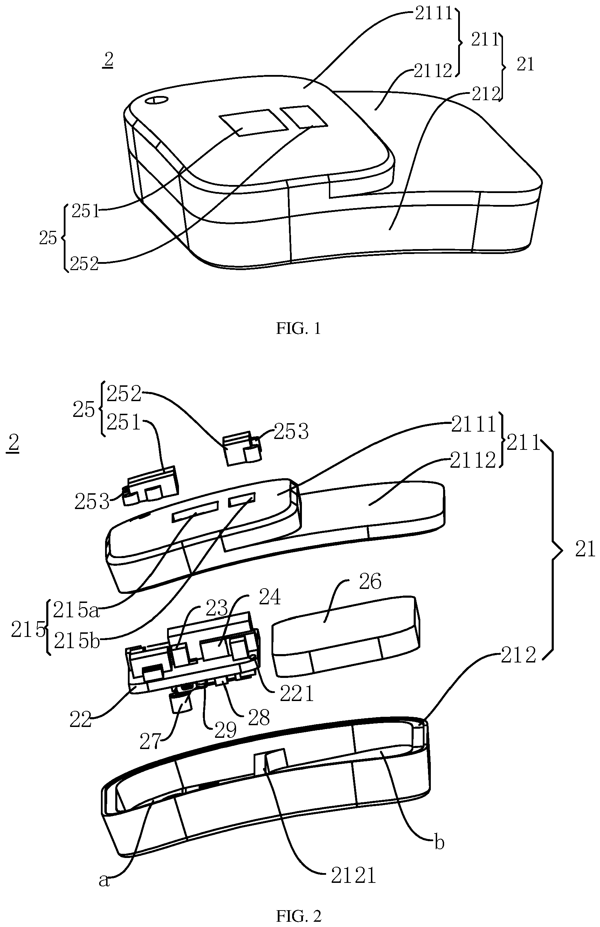

As shown in FIG. 1 and FIG. 2, the blood pressure measurement module 2 according to the embodiment of the present disclosure includes a housing 21, an emission element 23, a light receiver 24 and a circuit board 22.

Referring to FIG. 2, the housing 21 is provided with a holding space therein, and the housing 21 is provided with a transparent area thereon. The transparent area can be disposed on an outer surface of the housing 21. The circuit board 22 is disposed in the holding space. The emission element 23 and the light receiver 24 are both disposed on the circuit board 22.

In the related art, wearable devices basically adopt a connection mode of double-sided watch strap and middle watch body, in which the blood pressure measurement module is integrated in the middle watch body. However, due to a limited volume of the watch body, it is difficult to accommodate a large volume of health measurement module, and cannot achieve high-precision blood pressure measurement function.

In the present disclosure, the blood pressure measurement module 2 can be integrated into an independent module to facilitate the use of the blood pressure measurement module 2 by providing the housing 21 and making the circuit board 22, the emission element 23, the light receiver 24, etc. be disposed in the holding space inside the housing 21, so that a measurement position of the blood pressure measurement module 2 is not limited to a user's arm or wrist, and the measurement position is more flexible, it is beneficial to improve the accuracy of blood pressure measurement module 2.

At the same time, when the blood pressure measurement module 2 is applied to a wearable device, an installation position of the blood pressure measurement module 2 is also more flexible and diverse. For example, the blood pressure measurement module 2 in the present disclosure can be integrated on a watch strap 100 of the wearable device without occupying a space of a watch body of the wearable device, which not only saves an internal space of the watch body, but also does not need to be limited by the volume of the watch body, thus the blood pressure measurement module 2 can be enlarged in actual production, which is conducive to realizing the high-precision measurement function of the blood pressure measurement module 2.

The emission element 23 is used to transmit infrared light to a user's finger through the transparent area. For example, when using the blood pressure measurement module 2 to measure blood pressure, the user can press a pulp of one of fingers on the transparent area, the emission element 23 can emit the infrared light towards the user's pulp through the transparent area. Because capillaries in the finger pulp of the user are more abundant than those in the arm or wrist, a blood pressure value of the user can be accurately measured.

The light receiver 24 is electrically connected with the emission element 23. The light receiver 24 is used to receive the infrared light reflected by the user's finger through the transparent area. The circuit board 22 is used to calculate the blood pressure value based on the reflected infrared light received by the light receiver 24. Specifically, the circuit board 22 can be integrated with a sensor for calculating the blood pressure value according to the infrared light received by the light receiver 24, and the sensor is electrically connected with the light receiver 24.

For example, when the blood pressure measurement module 2 of the embodiment is used for blood pressure measurement, the user can press the transparent area with his finger, and the emission element 23 transmits infrared light through the transparent area towards the finger pressed on the transparent area. The infrared light is transmitted to subcutaneous vascular tissues of the user through the transparent area, and then the infrared light diffusely reflected by the blood is received by the light receiver 24, the sensor on the circuit board 22 calculates the blood pressure value of the user according to the reflected infrared light received by the light receiver 24.

It should be noted that because the content of oxygen-carrying hemoglobin and reducing hemoglobin in the blood is changing during human respiration, an absorption coefficient of blood to light is also changing, so a pulse wave curve that changes with the fluctuation of respiratory movement can be obtained. Then, an acceleration pulse wave curve is obtained after the second derivative of the pulse wave curve. The accelerated pulse wave curve can well reflect the process of blood microcirculation. Then feature points in the accelerated pulse wave curve can be extracted, generally the maximum point, the minimum point, a middle zero point and middle extreme points are extracted. Finally, according to the above feature points and a pre-established blood pressure model, the blood pressure value of the user is calculated.

According to the blood pressure measurement module 2 in the embodiment of the present disclosure, the blood pressure measurement module 2 is integrated into an independent module to facilitate the use of the blood pressure measurement module 2, so that a measurement position of the blood pressure measurement module 2 is not limited to a user's arm or wrist, and a user's finger can be used as the measurement position, which is conducive to improving the measurement accuracy of the blood pressure measurement module 2. At the same time, when the blood pressure measurement module 2 is applied to a wearable device, an installation position of the blood pressure measurement module 2 is also more flexible and diverse. For example, the blood pressure measurement module 2 in the present disclosure can be integrated on the watch strap 100 of the wearable device without occupying a space of a watch body of the wearable device, which not only saves the internal space of the watch body, but also does not need to be limited by the volume of the watch body, thus the blood pressure measurement module 2 can be enlarged in actual production, which is conducive to realizing the high-precision measurement function of the blood pressure measurement module 2.

According to some embodiments of the present disclosure, the blood pressure measurement module 2 further includes a communication circuit 28, which is disposed on the circuit board 22 and used to send measurement data to other devices. For example, the communication circuit 28 can be used to transmit the measurement data to an electronic device such as a wearable device. The communication circuit 28 can be Bluetooth circuit, etc. The wireless communication between the blood pressure measurement module 2 and the wearable device can be realized by providing the communication circuit 28, which is convenient for data transmission between the blood pressure measurement module 2 and the wearable device.

According to some embodiments of the present disclosure, the transparent area is formed by a lens 25 embedded on the housing 21. The lens 25 has a high light transmittance. The infrared light emitted by the emission element 23 can be transmitted to the subcutaneous vascular tissue of the user through the lens 25, and the infrared light reflected from the subcutaneous vascular tissue of the user can be transmitted to the light receiver 24 through the lens 25. Therefore, the accuracy and efficiency of measurement can be further improved.

It can be understood that in other embodiments of the present disclosure, a part of the housing 21 can be set as a transparent part to form the transparent area, at which the transparent area and the housing 21 are integrated into a whole. The infrared light emitted by the emission element 23 can be transmitted to the user's finger through the transparent area, and the light reflected by the user's finger can be transmitted to the light receiver 24 through the transparent area, the structure is simple and the processing is convenient.

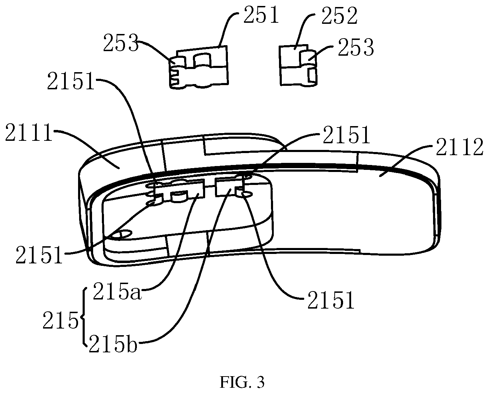

According to some embodiments of the present disclosure, as shown in FIG. 3, the lens 25 includes a first lens 251 and a second lens 252, the first lens 251 is facing towards the emission element 23, and the second lens 252 is facing towards the light receiver 24. The first lens 251 and the second lens 252 may be spaced apart on the housing 21. In an illustrated embodiment, the first lens 251 is facing towards the emission element 23 squarely, and the second lens 252 is facing towards the light receiver 24 squarely. That is, an orthographic projection of the first lens 251 on the circuit board 22 falls on the emission element 23, and an orthographic projection of the second lens 252 on the circuit board 22 falls on the light receiver 24. Therefore, the function of lens 25 is further optimized to improve the accuracy and efficiency of measurement.

According to some embodiments of the present disclosure, the lens 25 and the housing 21 are integrated into one body by injection molding. Therefore, the connection reliability of the lens 25 and the housing 21 can be improved, the processing of the blood pressure measurement module 2 can be simplified, and the processing cost can be reduced. For example, during the processing, the housing 21 can be formed by injection molding, and then the lens 25 can be embedded on the housing 21 by in-mold two-color injection molding. The process is simple and the processing is convenient.

According to some embodiments of the present disclosure, the housing 21 is provided with an inlaying groove 215, and the lens 25 is embedded in the inlaying groove 215. As shown in FIG. 3, the inlaying groove 215 is formed into a through groove that penetrates the housing 21 in a thickness direction of the housing 21. The inlaying groove 215 can include a first sub-groove 215a and a second sub-groove 215b, the first lens 251 is embedded in the first sub-groove 215a, and the second lens 252 is embedded in the second sub-groove 215b.

One of an outer peripheral wall of the lens 25 and an inner wall of the inlaying groove 215 is provided with a positioning protrusion 253, and the other of the outer peripheral wall of the lens 25 and the inner wall of the inlaying groove 215 is provided with a positioning groove 2151 which is matched with the positioning protrusion 253. Therefore, the connection reliability between the lens 25 and the housing 21 can be further improved.

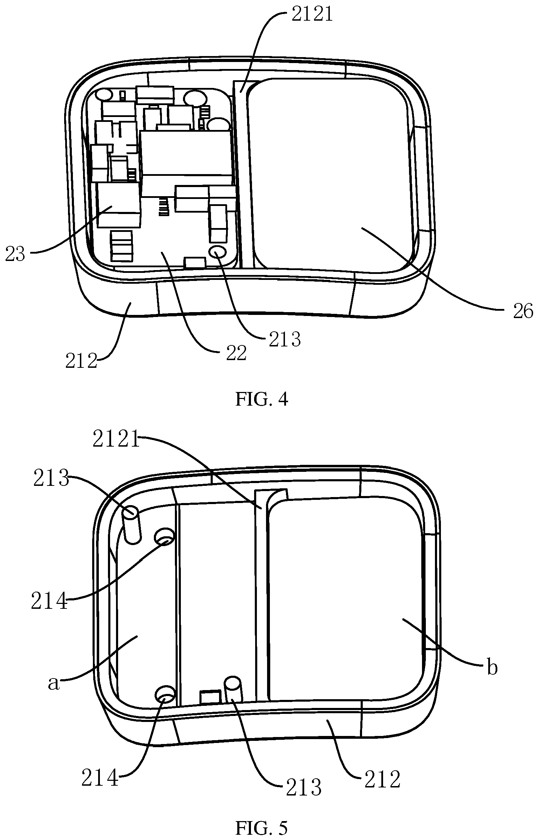

Specifically, when the positioning protrusion 253 is disposed on the outer peripheral wall of the lens 25, the positioning groove 2151 matching with the positioning protrusion 253 is disposed on the inner wall of the inlaying groove 215. When the positioning groove 2151 is disposed on the outer peripheral wall of the lens 25, the inner wall surface of the inlaying groove 215 is provided with the positioning protrusion 253 matching with the positioning groove 2151. For example, as shown in FIG. 4, the positioning protrusions 253 are formed on the outer peripheral wall of the lens 25, and the positioning grooves 2151 are defined on the inner wall of the inlaying groove 215. Each of the positioning protrusions 253 can be formed into a semicylindrical shape, the structure is simple and the processing is convenient.

According to some embodiments of the present disclosure, the housing 21 is provided with a first positioning structure 213, and the circuit board 22 is provided with a second positioning structure 221 that is matched with the first positioning structure 213. In some embodiments of the present disclosure, the first positioning structure 213 is a positioning column formed on the housing 21, and the second positioning structure 221 is a positioning hole defined on the circuit board 22. The structure is simple, the positioning is convenient and the processing is easy.

Of course, it can be understood that in other embodiments of the present disclosure, the first positioning structure 213 can also be a snap formed on the housing 21, and the second positioning structure 221 is a snap slot defined on the circuit board 22, which is used to position the circuit board 22 through the combination of the snap and the snap slot. Therefore, the positioning of circuit board 22 can also be realized conveniently.

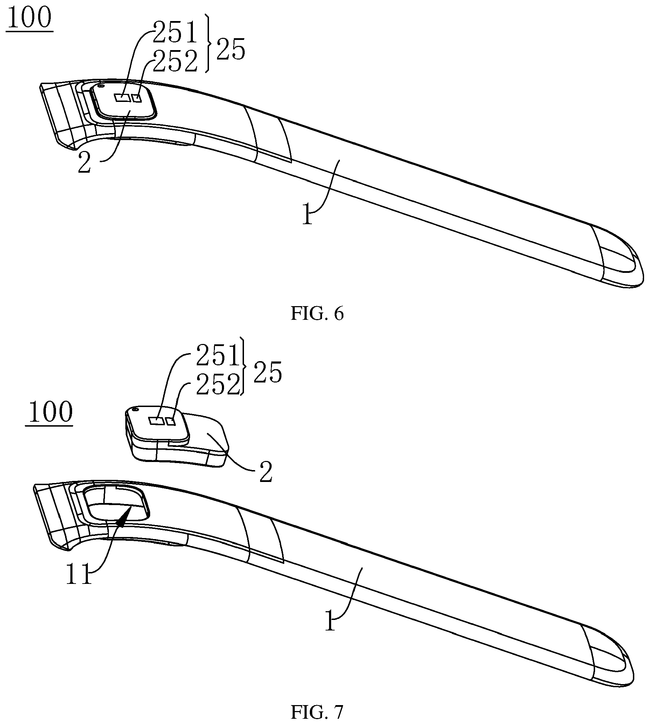

According to some embodiments of the present disclosure, as shown in FIGS. 4 and 5, the housing 21 is provided with a partition plate 2121, which divides the holding space into a first space a and a second space b, the circuit board 22 is disposed in the first space a, and the second space b is provided with a power supply 26 for supplying power to the circuit board 22. As shown in FIGS. 5 and 6, the partition plate 2121 can extend along a width direction or a length direction of the housing 21. The circuit board 22 and the power supply 26 can be separated by the partition plate 2121, which effectively prevents the heat generated by the power supply 26 from being transferred to the first space a, thus avoiding the impact of the above heat on components on the circuit board 22 and improving the reliability of the blood pressure measurement module 2.

In an illustrated embodiment, the power supply 26 includes a battery. The power supply 26 can be bonded in the second space b. For example, referring to FIG. 5, the power supply 26 can be bonded in the second space b by double-sided adhesive tape or the like. Therefore, when using the health measurement module, there is no need to connect the external power supply, which improves the convenience of using the blood pressure measurement module 2.

According to some embodiments of the present disclosure, the housing 21 includes a first housing 211 and a second housing 212, and the first housing 211 is connected with the second housing 212. Referring to FIG. 3 and in combination with FIGS. 4 and 5, the first housing 211 includes a first part 2111 and a second part 2112, the first space a is defined between the first part 2111 and the second housing 212, the second space b is defined between the second part 2112 and the second housing 212, the first part 2111 protrudes towards a direction facing away from the second housing 212 to form an avoidance space, and the second part 2112 is formed into a plate-type structure. By making the first part 2111 of the first housing 211 protrude towards the direction facing away from the second housing 212 to form the avoidance space, an accommodation space of the first space a is increased, and the interference of the inner wall of the housing 21 with the circuit board 22 is avoided. At the same time, by making the second part 2112 of the first housing 211 as the plate-type structure, the overall volume of the housing 21 can be reduced, the occupied space of the blood pressure measurement module 2 can be reduced, and the appearance aesthetics of the housing 21 can be improved.

According to some embodiments of the present disclosure, the circuit board 22 is provided with a charging connector 27, of which, the charging connector 27 can be two, and the two charging connectors 27 are spaced apart in a length direction of the circuit board 22. A bottom wall of the housing 21 is provided with through grooves 214, and the charging connectors 27 extend into the through grooves 214 respectively and are connected with the external power supply to charge the power supply 26.

Specifically, referring to FIG. 2, the charging connector 27 can be disposed on a lower surface of the circuit board 22. An end of the charging connector 27 is connected with the circuit board 22, and another end of the charging connector 27 can be electrically connected with the external power supply. For example, in some embodiments of the present disclosure, the charging connector 27 is connected with the circuit board 22 through a resilience sheet 29. When charging the power supply 26, the charging connector 27 can be in contact with the external power supply. Under the contact effect of the external power supply, the charging connector 27 can move towards a direction close to the circuit board 22 and compress the resilience sheet 29. Thus, the charging connector 27 and the circuit board 22 can be kept in better contact to ensure the smooth charging process.

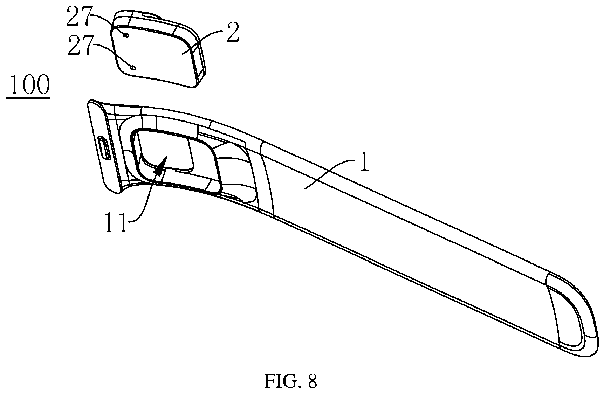

As shown in FIGS. 6 to 8, a watch strap 100 for wearable devices according to the second aspect of the present disclosure includes: a strap body 1 and the blood pressure measurement module 2 according to the above embodiments of the present disclosure, and the strap body 1 is provided with an installation groove 11. The blood pressure measurement module 2 is disposed in the installation groove 11.

Referring to FIGS. 6 to 8, the installation groove 11 can be a through groove 214 penetrating the strap body 1 in a thickness direction of the strap body 1, and the blood pressure measurement module 2 can be installed in the installation groove 11. By making the blood pressure measurement module 2 be disposed on the strap body 1, it does not occupy the space of the watch body of the wearable device, which can not only save the internal space of the watch body, but also does not need to be limited by the volume of the watch body, thus the blood pressure measurement module 2 can be enlarged in actual production, which is conducive to achieving the high-precision blood pressure measurement function of the wearable device.

In some embodiments of the present disclosure, the blood pressure measurement module 2 is detachably arranged in the installation groove 11. Therefore, the blood pressure measurement module 2 can be easily assembled and disassembled, thus facilitating the later maintenance of the blood pressure measurement module 2.

For example, in some embodiments of the present disclosure, the blood pressure measurement module 2 and the installation groove 11 have an interference fit. During assembly, the blood pressure measurement module 2 can be squeezed into the installation groove 11 from an end of the installation groove 11. During disassembly, the blood pressure measurement module 2 can be extruded from the installation groove 11 from an end of the installation groove 11. The structure is simple and the disassembly and assembly is convenient.

Of course, it can be understood that the cooperation between the blood pressure measurement module 2 and the installation groove 11 in the present disclosure is not limited to this. For example, in other embodiments of the present disclosure, the blood pressure measurement module 2 and the installation groove 11 can be connected and matched through a snap structure, and the disassembly and assembly of the blood pressure measurement module 2 can also be realized conveniently.

According to the embodiment of the present disclosure, the watch strap 100 for wearable devices is provided with the installation groove 11 on the strap body 1, and the blood pressure measurement module 2 is installed in the installation groove 11, so that the blood pressure measurement module 2 does not need to be limited by the volume of the watch body, the health measurement module can be enlarged in actual production, which is conducive to achieving the high-precision health measurement function of wearable devices. At the same time, the blood pressure measurement module 2 is integrated into an independent module, which is convenient for the use of the blood pressure measurement module 2, the measurement position of the blood pressure measurement module 2 is not limited to the user's arm or wrist, and the user's finger can be used as the measurement position, which is conducive to improving the measurement accuracy of the blood pressure measurement module 2.

According to some embodiments of the present disclosure, the housing 21 includes a first surface and a second surface disposed facing towards the first surface in a thickness direction of the strap body 1. The first surface and the second surface are both formed into arc-shaped surfaces, and a radian of the first surface is consistent with a radian of an outer surface of the strap body 1, and a radian of the second surface is consistent with a radian of an inner surface of the strap body 1. Thus, the integrity and appearance of the watch strap 100 can be improved, and the warping function of the watch strap 100 can be guaranteed.

According to the embodiment of the present disclosure, the watch strap 100 for wearable devices is embedded with the blood pressure measurement module 2 in the installation groove 11 of the watch strap 100, which can measure the user's blood pressure efficiently and accurately. The watch strap 100 is simple in assembly and compact in appearance, and the warping function of the watch strap 100 is not affected.

A wearable device according to an embodiment of a third aspect of the present disclosure includes a watch body and the watch strap 100. The watch strap 100 is the watch strap 100 according to the embodiments of the second aspect of the present disclosure, and the watch strap 100 is connected with the watch body.

In some embodiments of the present disclosure, the installation groove 11 is disposed on an end adjacent to the watch body of the strap body 1. Therefore, it is convenient for users to use the blood pressure measurement module 2.

The wearable device according to the embodiment of the third aspect of the present disclosure, by providing the watch strap 100 according to the embodiment of the second aspect of the present disclosure, and providing the installation groove 11 on the strap body 1, and installing the blood pressure measurement module 2 in the installation groove 11, the blood pressure measurement module 2 does not need to be limited to the volume of the watch body, so that the blood pressure measurement module 2 can be enlarged during actual production, it is beneficial to realize the high-precision health measurement function of wearable devices. At the same time, the blood pressure measurement module 2 is integrated into an independent module, which is convenient for the use of the blood pressure measurement module 2, the measurement position of the blood pressure measurement module 2 is not limited to the user's arm or wrist, and the user's finger can be used as the measurement position, which is conducive to improving the measurement accuracy of the blood pressure measurement module 2.

In the description of the present disclosure, it is necessary to understand that the orientation or position relationship indicated by the terms "bottom", "inside", "outside", etc. is based on the orientation or position relationship shown in the attached drawings, which is only for the convenience of describing the present disclosure and simplifying the description, rather than indicating or implying that the device or element referred to must have a specific orientation, be constructed and operated in a specific orientation, so it cannot be understood as a limitation of the present disclosure. In the description of the present disclosure, "multiple" means two or more.

In the description of the present disclosure, the first feature "above" or "below" the second feature can include direct contact between the first and second features, or indirect contact between the first and second features through another feature between them.

In the description of the specification, the description of the reference terms "one embodiment", "some embodiments", "schematic embodiment", "example", "specific example", or "some examples" means that the specific features, structures, materials, or features described in combination with the embodiment or example are included in at least one embodiment or example of the present disclosure. In this specification, the schematic expressions of the above terms do not necessarily refer to the same embodiments or examples. Furthermore, the specific features, structures, materials or characteristics described may be combined in an appropriate manner in any one or more embodiments or examples.

Although embodiments of the present disclosure have been shown and described, those skilled in the art can understand that these embodiments can be varied, modified, replaced and amended without departing from the principles and purposes of the present disclosure. The scope of the present disclosure is limited by the claims and their equivalents.

## Claims

1. A blood pressure measurement module, comprising:
a housing, provided with a holding space therein and a transparent area thereon;
an emission element, a light receiver and a circuit board;
wherein the emission element and the light receiver are disposed on the circuit board, the circuit board is disposed in the holding space, the emission element is configured to transmit infrared light to a finger of a user through the transparent area; the light receiver is electrically connected with the emission element, the light receiver is configured to receive infrared light reflected by the finger of the user through the transparent area; and the circuit board is configured to calculate, based on the reflected infrared light received by the light receiver, a blood pressure value.

2. The blood pressure measurement module according to claim 1, further comprising: a communication circuit, disposed on the circuit board and configured to send measurement data to other devices.

3. The blood pressure measurement module according to claim 1 or 2, wherein the transparent area is formed by a lens embedded on the housing.

4. The blood pressure measurement module according to claim 3, wherein the lens comprises a first lens and a second lens, the first lens is facing towards the emission element, and the second lens is facing towards the light receiver.

5. The blood pressure measurement module according to claim 4, wherein the first lens and the second lens are spaced apart on the housing.

6. The blood pressure measurement module according to claim 3, wherein the lens and the housing are integrated into one whole by injection molding.

7. The blood pressure measurement module according to claim 6, wherein the lens is embedded on the housing by in-mold two-color injection molding.

8. The blood pressure measurement module according to claim 3, wherein the housing is provided with an inlaying groove, the lens is embedded in the inlaying groove, one of an outer peripheral wall of the lens and an inner wall of the inlaying groove is provided with a positioning protrusion, and the other of the outer peripheral wall of the lens and the inner wall of the inlaying groove is provided with a positioning groove matching with the positioning protrusion.

9. The blood pressure measurement module according to claim 8, wherein the inlaying groove comprises a first sub-groove and a second sub-groove, the first lens is embedded in the first sub-groove, and the second lens is embedded in the second sub-groove.

10. The blood pressure measurement module according to any one of claims 1-9, wherein the housing is provided with a first positioning structure, and the circuit board is provided with a second positioning structure matching with the first positioning structure.

11. The blood pressure measurement module according to claim 10, wherein the first positioning structure is a positioning column formed on the housing, and the second positioning structure is a positioning hole defined on the circuit board.

12. The blood pressure measurement module according to claim 10, wherein the first positioning structure is a snap formed on the housing, and the second positioning structure is a snap slot defined on the circuit board.

13. The blood pressure measurement module according to any one of claims 1-12, wherein the housing is provided with a partition plate therein, configured to divide the holding space into a first space and a second space, the circuit board is disposed in the first space, and the second space is provided with a power supply for supplying power to the circuit board.

14. The blood pressure measurement module according to claim 13, wherein the power supply is bonded in the second space.

15. The blood pressure measurement module according to claim 13, wherein the housing comprises a first housing and a second housing, the first housing is connected with the second housing; the first housing comprises a first part and a second part, the first space is defined between the first part and the second housing, and the second space is defined between the second part and the second housing; and the first part protrudes towards a direction facing away from the second housing to define an avoidance space, and the second part is formed into a plate-type structure.

16. The blood pressure measurement module according to claim 13, wherein the circuit board is provided with a charging connector, a bottom wall of the housing is provided with a through groove, and the charging connector extends into the through groove and is connected with an external power supply to charge the power supply.

17. A watch strap for wearable devices, comprising:
a strap body, provided with an installation groove thereon; and
the blood pressure measurement module according to any one of claims 1-16, disposed in the installation groove.

18. The watch strap for wearable devices according to claim 17, wherein the housing comprises a first surface and a second surface opposite to the first surface in a thickness direction of the strap body, the first surface and the second surface are both formed into arc-shaped surfaces, and a radian of the first surface is consistent with a radian of an outer surface of the strap body, and a radian of the second surface is consistent with a radian of an inner surface of the strap body.

19. The watch strap for wearable devices according to claim 17, wherein the installation groove is a through groove penetrating the strap body in a thickness direction of the strap body.

20. A wearable device, comprising:
a watch body; and
the watch strap according to any one of claims 17-19, connected with the watch body.
